# EUROPEAN PATENT APPLICATION

(11) **EP 1 547 527 A1**
(43) Date of publication of application: **29.06.2005**
(21) Application number: 03794291.9
(22) Date of filing: 09.09.2003
(51) Int. Cl.: A61B 17/00, A61B 17/22, A61B 17/12

(54) **STRIPPING WIRE AND STRIPPING CATHETER FOR EVULSING VEIN**

(30) Priority: 09.09.2002 JP 2002262659
(71) Applicant: Ninomiya, Junichi, Tokyo 153-0052 (JP); ACP Japan Co. Ltd., Tokyo 113-0033 (JP)
(72) Inventor: NINOMIYA, Junichi, Meguro-ku, Tokyo 153-0052 (JP); NAKAMURA, Shoichi, Iruma-gun, Saitama 354-0041 (JP)
(74) Representative: Gill, David Alan
(86) International application number: PCT/JP2003/011483
(87) International publication number: WO 2004/021891

(57) **Abstract**

To provide a stripping wire/catheter which enables to connect a plurality of wires or catheters to constitute a stripping wire/catheter having a fixed length, an insert head, a rear end portion or a connected portion being a vein ligating portion.

A stripping wire/catheter for removing a vein comprises a first wire/catheter having an insert head for guiding into a vein in one end thereof and first connecting means in the other end thereof; and a second wire/catheter having second connecting means that can be connected with the first connecting means in one end thereof and a rear end portion in the other end thereof, wherein there is formed a vein ligating portion in order that a connected portion having the first connecting means and the second connecting means connected, a connected portion having the first connecting means and the insert head connected, or a connected portion having the second connecting means and the rear end portion connected ligates and draws the vein to thereby introvert and remove the vein.

## Description

### TECHNICAL FIELD

The present invention relates to a stripping wire and stripping catheter for performing a surgical operation upon a vein or varicose veins.

### BACKGROUND ART

Blood plays a part of circulating within the human body to supply oxygen and nutriment to various parts of the human body and gathering carbon dioxide and waste matter from various parts of the human body. A blood vessel is an important organ bearing the circulation of blood, and is roughly classified into an artery and a vein.

The artery is a pipe for sending blood delivered from the heart to all over the body, and the vein is a pipe for returning blood sent to the all over the body to the heart.

For example, in the case of blood circulation of leg portions, blood delivered from the heart is sent to every nook and corner of the leg passing through an artery, and returns to a vein passing through the skin, muscles and the like. Blood entered a vein of the leg portion is necessary to move up from the leg toward the heart against the gravity, and a constant pressure is necessary. A vein valve and muscles of legs perform an important function for producing a fixed pressure in blood within the blood vessel.

The vein valve plays a part for preventing a backflow of blood to flow blood upward always, but the vein valve itself has no force for flowing blood positively, and muscles of the calf mainly function to extrude blood upward. When muscles of the calf contract, a vein of legs is oppressed to push up blood. In this manner, the vein valve and the pumping action of muscles cooperate so that blood of legs is carried to the heart.

A varicose vein is a disease such that a valve of a vein of legs fails to function properly, and blood brings forth a backflow or blood congestion to worsen the circulation of blood of legs. Since blood stays in a vein of legs, a vein becomes thick or meanders. The staying condition of blood becomes worsened, the skin becomes inflamed or the skin becomes light brown and cakes. Further, the skin necroses into ulcer which is difficult to recover.

Treatments for varicose veins include generally, an oppression treatment, a hardening treatment, and a surgical treatment. In case of slight illness, an oppression treatment or a hardening treatment is used for treatment, and in case of serious illness, a surgical treatment is carried out. The surgical treatment (stripping operation) is a surgical operation in which many small skin-incisions are provided to remove occlusion or varicosed varicose veins.

Japanese Unexamined Patent Publication No. 507714/1996 discloses a conventional device for the local extirpation of varicose veins for removing such a vein blood vessel as described above. The device for the local extirpation of varicose veins comprises a head part comprising a main body provided with barbs and a guiding tip facilitating the insertion of the device into the vein, the barbs being oriented backwards relative to the direction of insertion of the device so that the head part can be inserted into the vein without being impeded by the barbs. A flexible actuating means is attached to the head so that the vein can be gripped firmly by the barbs when the head is pulled out from the vein at an angle relative to the longitudinal direction of the head part in the operative position of the barbs.

However, the stripping wire for removing a vein according to the above-described prior art comprises a single wire, and therefore, it is too short or too long depending upon a part to be treated of varicose veins so that the surgical operation is in trouble. Therefore, it has been necessary to prepare stripping wires of various lengths adjusted to a part subjected to surgical operation.

Further, where a vein is removed by holding and firmly gripping the vein by the barbs, the vein is cut or damaged in its peripheral portions by the barbs during drawing it. Further, dregs of a vein torn remain in wound, and further the dregs have to be removed.

Moreover, in the treatment of a part from which a vein is removed after the removal of varicose veins, to press bleeding and stop bleeding by the palm on a travel line of a long saphenous vein (LSV) are being carried out. In the administration of a medicine into wound after removal of varicose veins, there poses a problem that it is necessary to administer a medicine on the affected part using a medicine administering device or the like.

It is an object of the present invention to provide a stripping wire for introverting a vein to remove it, wherein a plurality of wires can be connected to provide a stripping wire having a fixed length, and not only a connected part in which the wires are connected but also an insert head or a rear end portion of the stripping wire become a vein ligating portion.

It is a further object of the invention to provide a stripping catheter for introverting a vein to remove it and being provided with a tube for administering a medicine or the like.

### DISCLOSURE OF THE INVENTION

Therefore, the present invention provides a stripping wire for removing a vein, comprising: a first wire having an insert head for guiding said stripping wire into a vein in one end thereof, and first connecting means in the other end thereof; and a second wire having second connecting means that can be connected with said first connecting means in one end thereof, and a rear end portion in the other end thereof, wherein said first wire and said second wire are connected for use, and there is formed a vein ligating portion in order that a connected portion caused by said first connecting means and said second connecting means, said insert head or said rear end portion is engaged with the affected part within the vein to introvert and remove the vein.

The present invention further provides a stripping wire for removing a vein, said stripping wire having a plurality of wires connected, each of said connected wires comprising first connecting means provided on one end thereof and second connecting means provided on the other end thereof, wherein an insert head for guiding said stripping wire into a vein is connected to said first connecting means in one end of said connected wire, a rear end portion is connected to said second connecting means in the other end of said connected wire, and there is formed a vein ligating portion in order that a connected portion caused by said first connecting means and said second connecting means between the wires, a connected portion connected said first connecting means and an insert head, or a connected portion connected said second connecting means and a rear end portion is engaged with the affected part within the vein to introvert and remove the vein.

By the structure as described above, in the present invention, the wires can be connected, for use, while adjusting to the distance from the incision portion to the vein affected part, or the distance from the first incision portion for inserting the insert head into the vein to the second cut-open portion for taking out the insert head outside the vein, and therefore it is not necessary to prepare in advance stripping wires of various lengths. Further, since fixed connecting means is provided on each wire, it is possible to select and mount insert heads of various shapes prepared in advance.

The first connecting means is formed from a fixed shaped male screw or female screw, and the second connecting means is formed from a male screw or a female screw in engagement with the first mentioned male screw or female screw. The insert head is provided with the second connecting means so that the former is connected to the first connecting means of the wire. Similarly, the rear end portion is provided with the first connecting means so that the former is connected to the second connecting means of the wire.

The insert head has an extreme end in an inserting direction formed into a convergent dome shape. The insert head is in an olive shape for removing a remained vein broken during removing the vein by a Babcock method. The rear end portion is in a cylindrical shape whose diameter is larger than the wire, and there is formed an operating grip for pushing in or drawing out in a direction of this side the insert head inserted into a vein.

Thereby, in the present stripping wire, even if a vein is broken during introverting and removing a vein, the vein can be removed in the direction reversed to the introverting direction.

Incidentally, the stripping wire used is a twist wire of which outside diameter is 1.5 to 3.0 mm. And, the surface of the wire is applied with a coating of silicon resin or elastic synthetic resin. Further, the surface of the wire is provided with depth marks at fixed intervals.

Further, the surface of said wire in the vicinity of the insert head is formed with fine concavo-convexes. Thereby, the frictional force between the insert head and the inner surface of a vein is maintained to prevent a vein during introverting from being disengaged from the insert head.

The present invention further provides a stripping catheter for removing a vein, wherein said stripping catheter has a plurality of catheters connected, each of said catheters connected first connecting means provided on one end and second connecting means provided on the other end, an insert head for guiding said stripping catheter into a vein is connected to said first connecting means on one end of said connected catheter, a rear end portion is connected to said second connecting means on the other end of said connected catheter, there is formed a vein ligating portion in order that a connected portion caused by said first connecting means and said second connecting means between the catheters, said insert head or said rear end portion is engaged with the affected part within the vein to introvert and remove the vein.

Here, a tube (a tube hole) within said catheters connected extends through from the rear end portion to the insert head, and a pouring port for pouring a flowing medicine is provided in the rear end portion, and the medicine poured from the pouring port flows into a hypodermic tunnel after removal of a vein from a hole provided in the insert head or said connected portion. Further, a pouring port for pouring a flowing medicine is provided in the rear end portion, the catheter is formed with a plurality of holes from the tube extending through the interior to the circumferential surface of the catheter, and the medicine poured from the pouring port flows into a hypodermic tunnel after removal of a vein from the plurality of holes. To this end, the pouring port is formed so that a syringe for pouring a medicine is connected.

Here, a guide wire for guiding the catheter into a vein is introduced into the tube of the catheter connected. Supersonic wave irradiating means for irradiating supersonic waves to the affected part in a vein from a hole provided in the insert head or said connected portion is introduced into the tube within said catheter connected. Further, laser beam irradiating means for irradiating laser beams to the affected part in a vein from a hole provided in the insert head or the connected portion is introduced into the tube within the catheter connected.

Further, a pouring port for pouring a flowing medicine is provided in the insert head of the present stripping catheter, and after the insert head inserted into a vein is drawn outside the human body at a fixed place within the vein, the medicine is poured from the pouring port of the insert head. In this case, in the connected portion between the plurality of catheters, a closing portion for closing the tube extending through the catheter connected is formed, whereby different medicines can be poured from the medicine pouring port provided in the insert head and the medicine pouring port provided in the rear end portion, and at least, the two kinds of different medicines can be prevented from being mixed till they are poured into the affected part.

### BRIEF DESCRIPTION OF THE DRAWINGS

FIG. 1 shows a side view of the whole stripping wire in a first embodiment according to the present invention.
FIG. 2 shows an enlarged view of the whole side view having a wire and a wire connected and a connected portion in a second embodiment according to the present invention.
FIG. 3 shows an example of a side view of a stripping wire having an insert head and a rear end portion threaded to the connected part in the second embodiment.
FIG. 4 shows a view for explaining the operation for removing varicose veins of the lower extremity according to the stripping wire of the present invention.
FIG. 5 shows the state that the connected part between wires engages the inner surface of a vein to be introverted.
FIG. 6 shows a side view of the whole stripping catheter in a third embodiment according to the present invention.
FIG. 7 shows a first modified example of the third embodiment shown in FIG. 6.
FIG. 8 shows a second modified example of the third embodiment shown in FIG. 6.
FIG. 9 shows the state that a guide wire is introduced into a tube of a stripping catheter.
FIG. 10 shows the state that supersonic wave irradiating means or laser irradiating means is introduced into a tube of a stripping catheter.
FIG. 11 shows an example in which first connecting means and second connecting means are joined to a single catheter.

### BEST MODE FOR CARRYING OUT THE INVENTION

The embodiments of a stripping wire for removing a vein according to the present invention will be described in detail hereinafter with reference to the drawings.

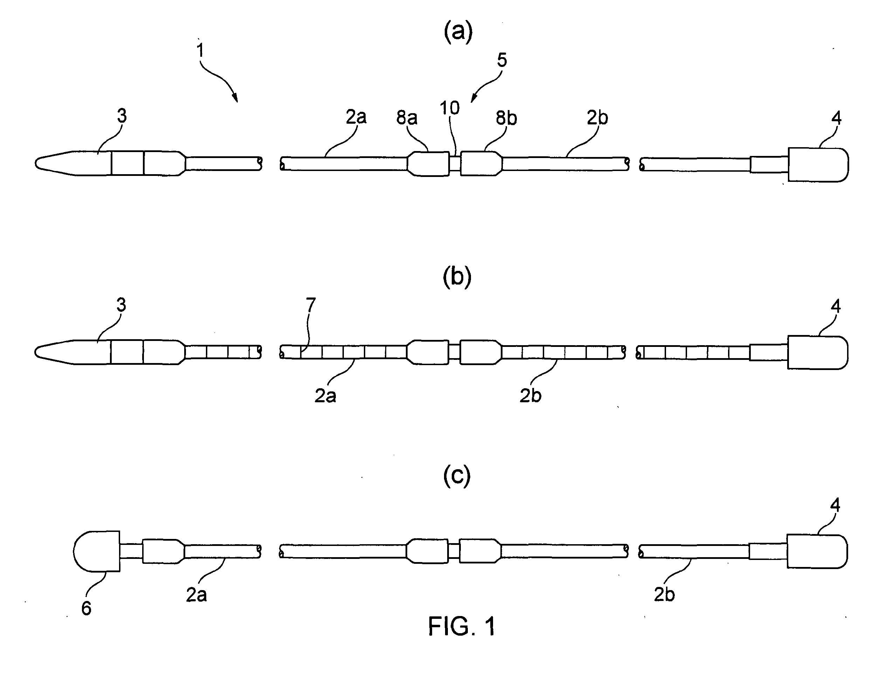

FIG. 1 shows the whole view of a stripping wire 1 in a first embodiment according to the present invention. In FIG. 1, the stripping wire 1 has a first wire 2a and a second wire 2b connected. The first wire 2a to be inserted into a vein is provided with an insert head 3 on one end (extreme end) thereof. The first wire 2a is provided, on the other end (rear end) thereof, with a connecting portion 8a (connecting means).

The second wire 2b is provided, on one end thereof, with a connecting portion 8b (connecting means) to be connected to the connecting portion 8a of the first wire 2a, and is provided, on the other end (rear end) thereof, with a rear end portion 4 gripping for operating the insertion of the stripping wire 1 into a vein.

The connecting portions 8a and 8b are joined or stopped by screw means for fitting a convex portion and a concave portion formed in the connecting portions 8a and 8b, fitting means such as a pawl, or chuck-shaped connecting means. Where screw means is used, the connecting portions 8a and 8b are connected by a male screw and a female screw formed and threaded in the connecting portions 8a and 8b.

As described, the connection of the connecting portion 8 of the present invention may be any connection by a fitting portion for fitting, or a stopping portion for stopping by a pawl or the like, or a screw portion to be connected by a male screw or a female screw.

In the description of the present embodiment, a description will be made of the connecting portions 8a and 8b as a male screw (first connecting means) and a female screw (second connecting means) formed and threaded in the connecting portions 8a and 8b.

The wires 2a and 2b are formed of a material which is flexible, elastic and semi-rigid (for example, such as stainless steel, polycarbonate, polyamide, polyoxymethylene, or the like). This stripping wire 1 is formed by connecting the connecting portion 8a provided in the first wire 2a and the connecting portion 8b provided in the second wire 2b. And, the connecting portions 8a and 8b in which the connecting portion 8a and the connecting portion 8b provided in the second wire 2b form a vein ligating portion 5 for ligating a vein.

However, alternatively, in the stripping wire 1, not only the joining portion 5 between wires, but also the insert head 3 or the rear end portion 4 can be engaged with the inner surface of a vein to provide the vein ligating portion introverting (turning over) a vein.

The treatment using the stripping wire 1 is carried out, for example, by applying skin incision to an inguinal portion, inserting it from the center portion of the long saphenous vein (LSV) toward the periphery, ligating and fixing the vein center end, drawing the stripping wire 1 gradually from the center toward the periphery, introverting the vein center end, and removing the vein from the knee incision wound.

The rear end portion 4 of the present stripping wire 1 is, in its diameter, cylindrical larger than the wire and forms an operating gripper for pressing the insert head inserted into the vein or pulling it our in the direction of this side. Thereby, the present stripping wire is able to invert and remove the vein in any direction.

Further, as the wires 2a and 2b, a twist wire having the outside diameter of 1.5 to 3.0 mm is used. Further, the surface of the wires 2a and 2b may be applied with coating of silicone resin or elastic synthetic resin.

Incidentally, the stripping wire 1 has an olive-shaped head 6 (removing head) mounted on the extreme end of the wire 2 in order to remove the remained vein broken caused by the stripping by a Babcock method. This olive-shaped head 6 is, when a vein to be removed is remained, attached to the connecting portion 8 of the wire 2 to gradually draw the stripping wire 1 from the center to the periphery to remove the remained vein.

In an example shown in FIG. 1 (a), the first wire 2a and the second wire 2b are provided with the connecting portions 8a and 8b formed with the male screw and the female screw, respectively. As shown in FIG. 1 (a), in the stripping wire 1, the first wire 2a and the second wire 2b are connected by screw means 10 formed with the connecting portion 8a (first connecting means) and the connecting portion 8b (second connecting means).

The connecting portions 8a and 8b having the first wire 2a and the second wire 2b connected form a vein ligating portion 5. The vein ligating portion 5 has a function of ligating a long saphenous vein to draw it toward the peripheral side thereby introverting a vein center end to remove it. And, the surface of the wire is coated with synthetic resin or the like to lower the frictional coefficient. Because of this, it is possible to prevent vein contraction from being generated at the time of inserting a wire.

FIG. 1 (b) shows a stripping wire 1 in which a depth marker 7 is formed on the surface of the first wire 2a and the second wire 2b so that the insert depth may be easily known. Other structures of the stripping wire 1 are similar to the structure shown in FIG. 1 (a).

In FIG. 1 (c), an olive-shaped head 6 is mounted on one end (extreme end) of the first wire 2a. The olive-shaped head 6 is provided for removing a remained vein broken by stripping during surgical operation by a Babcock method. Other structures of the stripping wire 1 are similar to the structure shown in FIG. 1 (a).

A description will be made of the structure of a plurality of wires 2 in which connecting portions are formed on both ends and the wire 2 for connecting the wires 2 to form a stripping wire 1, which is a second embodiment of the present invention.

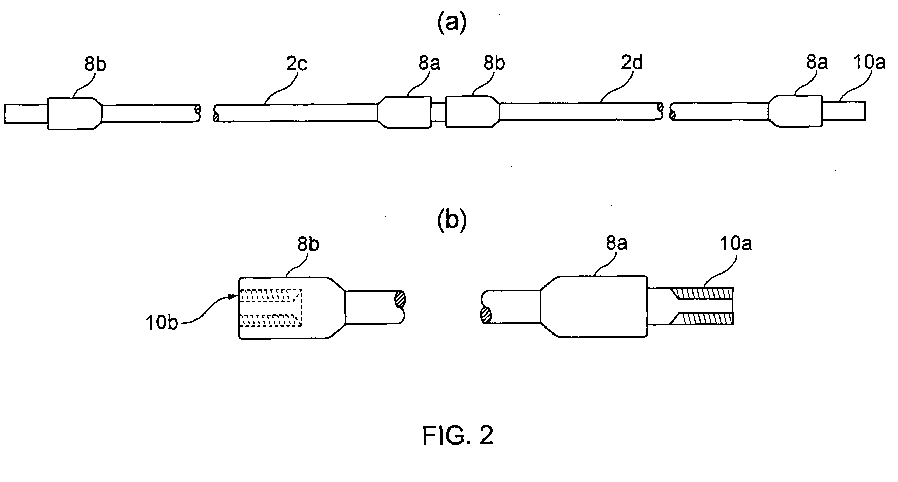

FIG. 2 shows a stripping wire according to a second embodiment of the present invention. In the second embodiment of the present invention, different from the first embodiment described previously, first connecting means 8a and second connecting means 8b are provided on both ends of the wire 2 for connecting a plurality (more than two) of wires 2.

FIG. 2 (a) shows the state that two wires 2c and 2d formed with the connecting portions 8a and 8b on both ends are connected. The connecting portion 8a formed with a male screw 10a is provided on one end of the wire 2c, and the connecting portion 8b formed with a female screw 10b is provided on the other end of the wire 2c. Further, the connecting portion 8b formed with a female screw 10b is provided on one end of the wire 2d, and the connecting portion 8a formed with a male screw 10a is provided on the other end of the wire 2d.

In FIG. 2 (a), the wires 2c and 2d are connected by threading the screws 10a and 10b formed in the connecting portions 8a and 8b. Further, the wires 2c and 2d are designed so that a necessary number of wires are connected such that the connecting portions 8 of another wire 2 are connected one after another to the connecting portions 8a till the desired fixed length is obtained.

As described above, a plurality of wires 2 can be connected one after another to have the fixed length by the connecting portions 8a and 8b in which the screws 10 provided on both ends of the wire 2 are formed.

FIG. 2 (b) shows connecting portions 8a and 8b provided on the wire 2d. The connecting portions 8b is formed with a female screw 10b, and the connecting portions 8a is formed with a male screw 10a.

In FIG. 2, the wire 2c and the wire 2d are connected by threading the male screw of the connecting portion 8a of the wire 2c and the female screw of the wire 2d. Further, for extending the wire 2, connection can be achieved, for example, by threading the connecting portion 8 having the female screw 10b of the other wire 2 in the connecting portion 8a of the wire 2d.

In this manner, the plurality of wires 2 can be connected one after another to provide the stripping wire 1 having a fixed length. However, it is possible that wires 2 having plural kinds of length are prepared, the wire 2 having a fixed length is selected adjusting to a part subjected to surgical operation, and the selected wire 2 is connected for use.

While in the present embodiment, in connecting of the connecting portions 8a and 8b, the screw 10 is used, it is noted that there may be employed a method for fitting or engaging the connecting portions 8a and 8b by means of a fitting member instead of the screw 10.

Further, in the stripping wire 1 of the present invention, a position of the connecting portion connected by the connecting portion 8 of the wire 2 is made to be a vein ligating portion 5. By connecting a plurality of wires 2, a plurality of vein ligating potions 5 are formed on the stripping wire 1.

With this, when the extreme end of the stripping wire 1 inserted from the center end assumes a skin incision position of the lowest varicose end, the vein ligating portion 5 at a position nearest to the uninserted stripping wire 1 can be used to ligate a vein.

In the conventional stripping wire 1, since the vein ligating portion 5 is provided approximately in the central portion of the stripping wire 1, it has been necessary to effect insertion till the vein ligating portion 5 assumes the ligating position of the center end for ligating a vein. In the stripping wire 1 of the present invention, a vein can be removed in the state that the vein ligating portion 5 in the vicinity of the center end (ligating position) is .used to ligate and draw the vein to introvert (invert) a blood vessel.

Further, in the stripping wire 1, where the length is short even during insertion and even during removal of a vein, the wire 2 is connected to enable extending the length.

Further, in the stripping wire according to the second embodiment, the insert head of various shapes and sizes prepared in advance is connected to the connecting portion 8b at the extreme end of the wire 2c, and the rear end portion of various shapes and sizes prepared in advance is connected to the connecting means 8a at the rear end of the wire 2d whereby a stripping wire adjusted to the inside diameter of a vein to be introverted can be obtained.

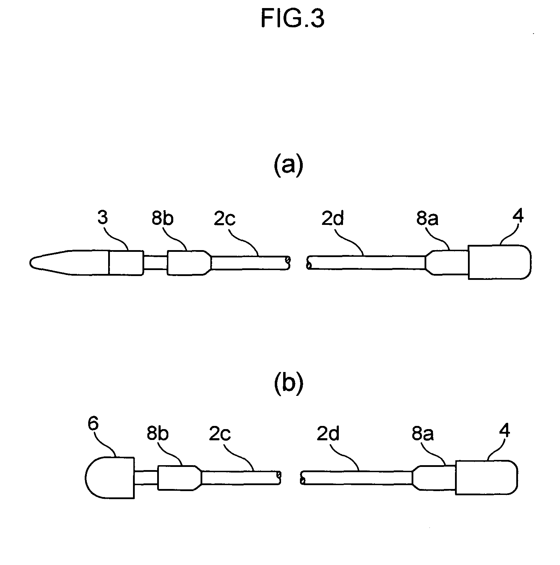

FIG. 3 (a) shows an example of a stripping wire 1 in which an insert head 3 and a rear end portion 4 are threaded and connected to a wire 2 formed with connecting portions 8 on both ends, which is a second embodiment. The insert head 3 is threaded by means of a screw to a connecting portion 8b on one end (extreme end) of a wire 2c. The rear end portion 4 is threaded to a connecting portion 8a at the rear end of a wire 2d.

FIG. 3 (b) shows an example of a stripping wire 1 in which an olive-shaped head 6 and a rear end portion 4 are threaded and connected to a wire 2 formed with connecting portions 8 on both ends, which is a second embodiment. The olive-shaped head 6 is threaded by means of a screw to one side (extreme end) of a wire 2c. The rear end portion 4 is threaded to a connecting portion 8a at the rear end of a wire 2d.

Since a screw is used for mounting, the insert head 3, the olive-shaped head 6, and the rear end portion 4 can be mounted positively by simple operation to one end (extreme end) of the wire 2c and the connecting portion on the other end of the wire 2d.

While in FIG. 3, a description has been made of the form in which the insert head 3 and the rear end portion 4 are threaded in the wire 2, it is to be noted as a separate form that in the wire 2c at the extreme end and the wire 2d at the rear end in the inserting direction into a vein, the first wire 2a and the second wire 2b (FIG. 1 (a)) to which the insert head 3 and the rear end portion 4 have been already fixed to thereby realize the present second embodiment.

A fixed number of wires 2c or wires 2d formed with the connecting portions 8 on both ends are connected between the first wire 2a and the second wire 2b to enable obtaining a stripping wire 1 having a fixed length.

The operation of an example for removing varicose veins of the lower extremity according to the stripping wire 1 of the present invention will be described hereinafter.

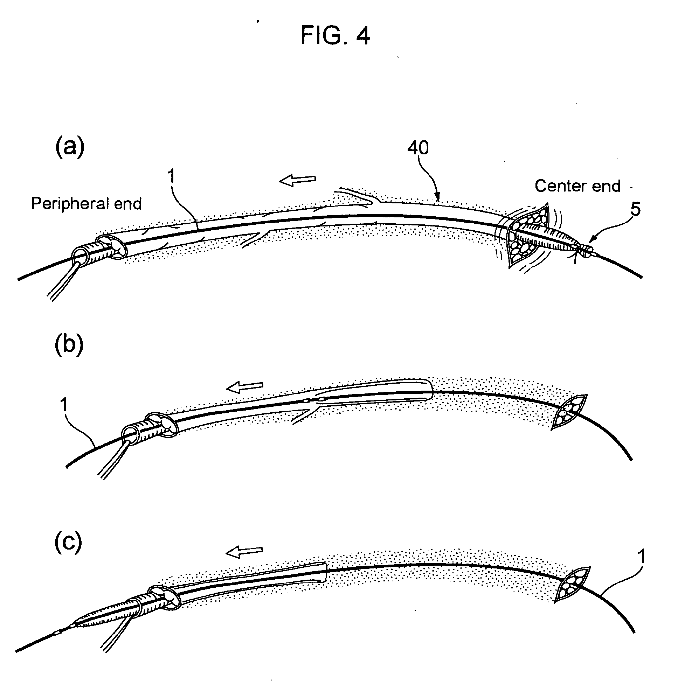

FIG. 4 shows a view for explaining the operation of an example for removing varicose veins of the lower extremity according to the stripping wire 1. The surgical operation treating method (stripping surgical operation) of varicose veins is a surgical operation treatment in which many small skin incisions are placed to remove expanded or varicosed varicose veins. The surgical operation treating method for removing varicose veins of the lower extremity is carried out in the following operation.
1. First, the inguinal portion is subjected to the skin incision from 1.5 cm to 2.0 cm to expose a long saphenous vein (LSV) 40.
2. The stripping wire 1 is inserted in a direction from the center end of the long saphenous vein 40 to the periphery (in a direction indicated by arrow in the figure) to fix the vein to the stripping wire 1 in one ligation.
3. The stripping wire 1 is moved to the varicose lowest end of the trunk, and small skin incision (peripheral end) is applied to the part sensitive to the insert head 3 of the stripping wire 1, or directly above a protuberance of the skin caused by pushing up the extreme end.
4. After the peripheral-side vein of the long saphenous vein 40 secured by the small kin incision has been exposed, the stripping wire 1 is drawn out of the vein.
5. As shown in FIG. 4 (a), the stripping wire 1 inserted into a vein is further drawn to the peripheral end side (in a direction indicated by arrow) to ligate and fix the vein center end to the vein ligating portion 5 in the vicinity of the center end of the stripping wire 1.
6. As shown in FIG. 4 (b), the stripping wire 1 is gradually drawn from the center to the periphery (in a direction indicated by arrow) to introvert the center end of a vein.
7. As shown in FIG. 4 (c), the stripping wire 1 is further drawn (in a direction indicated by arrow) to remove it from the small skin incision wound (peripheral end).

Where a breakage of a vein occurs during treatment of removal of a vein, the stripping wire 1 is drawn to the periphery as it is to remove the broken vein from the small skin incision wound, after which the vein peripheral end is ligated and fixed to the vein ligating portion 5, and the introverting removal (stripping) from the periphery to the center is carried out.

Further, where it is broken even in stripping from the periphery, the olive-shaped head 6 is mounted on the stripping wire 1 at the center position, and the remained vein is drawn toward the periphery to remove it by the Babcock method. Further, after the completion of the removal of a vein, oppression hemostasis operation for about 5 minutes is carried out by the pawl on the long saphenous vein travel line.

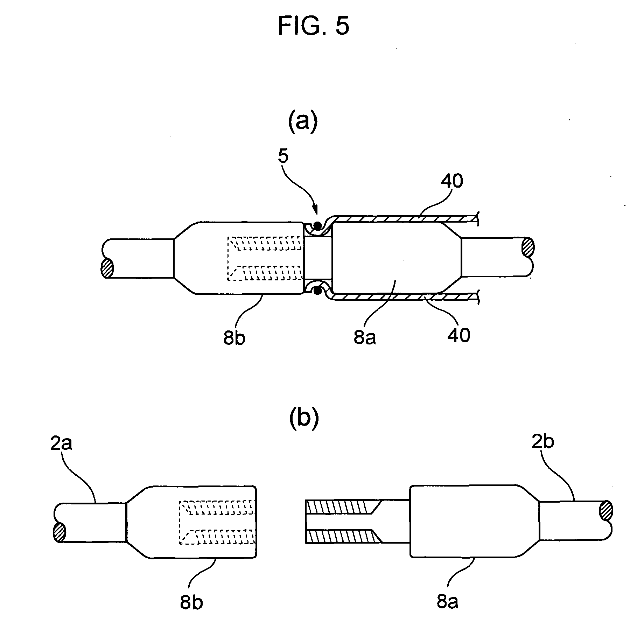

FIG. 5 shows the state that the connecting portions 8a and 8b between the wires engage the inner surface of the introverting vein.

FIG. 5 (a) shows the state that the connecting portions 8a and 8b between the wires constitute the vein ligating portion 5 and engage the inner surface of the introverting vein. Since the vein has elasticity, it engages a depressed portion between the first connecting means 8a and the second connecting means 8b so that the engaged portion becomes caught on the inner wall of a vein to constitute the vein ligating portion 5.

As shown in FIG. 5 (b), the second connecting means (female screw) 8b connected to the rear end side of the first wire 2a is connected to the first connecting means (male screw) 8a connected to the extreme end side of the second wire 2b. Thereby, the connecting portion between the wires constitutes the vein ligating portion 5.

A third embodiment of the present invention will be described hereinafter.

The interior of the vein removed wound after introversion becomes damaged and bleeds. Therefore, in the treatment, gauze is firmly stuffed into the removed wound to press and stop blood, and pressing and stopping blood is carried out on the long saphenous vein travel line by the palm.

However, for the early recovery and treatment of the removed wound, a medicine is administered into the wound (affected part) by means of a medicine administering device or the like, and a laser beam or a supersonic wave can be irradiated to thereby treat the vein removed wound early to stop blood.

In the third embodiment of the present invention, a stripping catheter is used in place of a stripping wire. The stripping catheter is that the center shaft of the stripping wire in the second embodiment of the present invention described previously is a hollow tube.

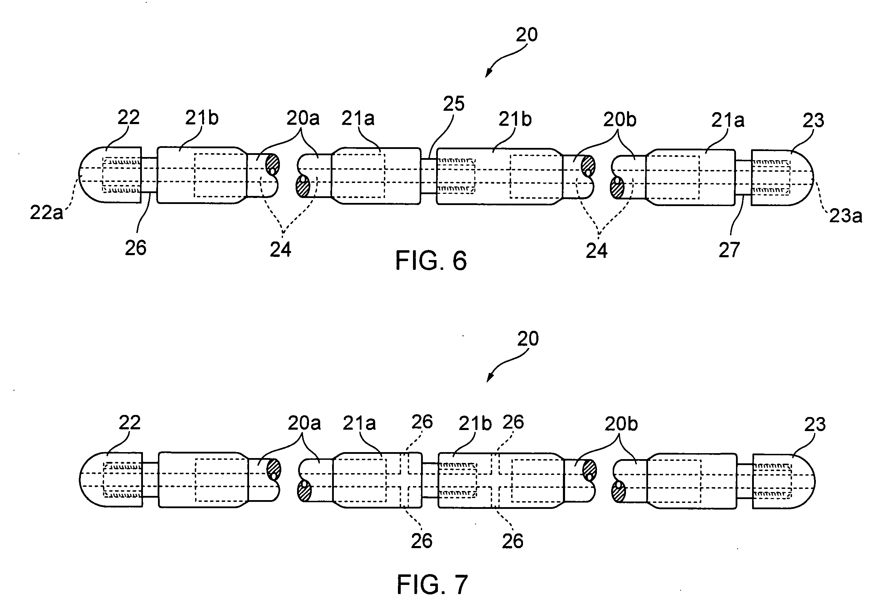

Fig. 6 shows the whole view of the present stripping catheter.

The present stripping catheter 20 consists of a first catheter 20a and a second catheter 20b connected each other. The two stripping catheters are that first connecting means 21a of the first catheter 20a and second connecting means 21b of the second catheter 20b are connected to constitute a connecting portion 25. The connecting portion 25 constitutes the vein ligating portion 5. Further, alternatively, a depression formed between the first catheter 20a and the connecting means at the extreme end thereof, and the insert head 22, and a depression formed between the second catheter 20b and the connecting means at the rear end thereof, and the rear end portion 23 may likewise constitute the vein ligating portion.

In the stripping catheter 20 shown in FIG. 6, a tube 24 within the first catheter 20a and a tube 24 within the second catheter extend through from the insert head 22 to a connecting portion 25 and from the connecting portion 25 to the rear end portion 23. This enables that a medicine poured from a medicine pouring port 23a of the rear end portion 23 flows out of an outflow port 22a provided at the extreme end of the insert head 22 and is administered to the affected part. Therefore, a medicine poring syringe is connected to the rear end portion 23.

Further, the present stripping catheter 20 can be also removed in the direction reversed to the inserting direction of the insert head 22, and so, the outflow port 22a provided at the extreme end of the insert head 22 may be also used as a flowing medicine pouring port. That is, after the insert head 22 inserted into a vein has been pulled outside the human body at a fixed place within the vein, a medicine is poured from the pouring port (outflow port) 22a of the insert head.

Incidentally, the present stripping catheter 20 is formed of a material which has flexibility and elasticity, and semi-rigidity (for example, such as nylon, PTFE, polyimide, polyurethane, and stainless).

FIG. 7 shows a first modified form of the third embodiment shown in FIG. 6. Also in the connecting means 21a and 21b of the present stripping catheter 20, outflow holes from which a medicine flows out are provided. In the flowing medicine poured from the rear end portion 23, since a medicine flows out of also the connecting means 21a and 21b in the midst to the insert head 22, a medicine can be widely flown into a hypodermic tunnel when a vein is removed. Because of this, a medicine outflow hole for communicating a tube with the outer circumferential surface of a catheter is applied to the catheter itself, whereby after removal of a vein, a medicine can be also administered in the wide range in the hypodermic tunnel.

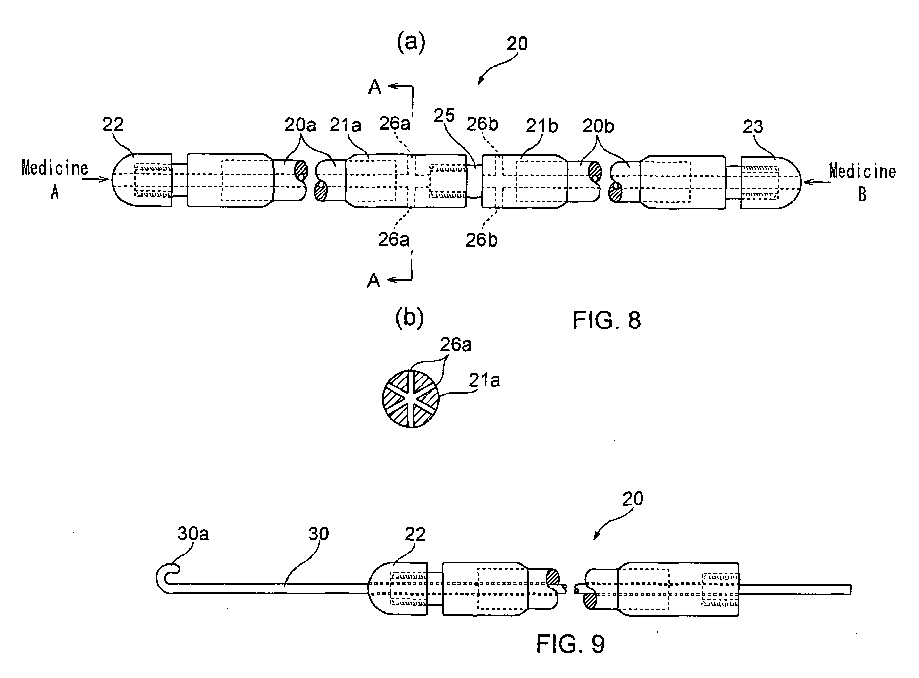

FIG. 8 is a second modified form of the third embodiment shown in FIG. 6, showing an example of a stripping catheter in which in a connecting portion 25 between a first catheter 20a and a second catheter 20b, a closing portion for closing a tube extending through the connected catheter is formed. Thereby, a medicine poured from the insert head 22 can be made different from a medicine poured from the rear end portion 23, and at least, the two kinds of different medicines can be prevented from being mixed till it is poured into the affected part. As shown in FIG. 8, a medicine A poured from the insert head 22 flows out of a medicine outflow port 26a provided in first connecting means 21 a in a first catheter 20a, and a medicine B poured from the rear end portion 23 flows out of a medicine outflow port 26b provided in second connecting means 21b in a second catheter 20b.

FIG. 9 shows the state that a guide wire 30 is inserted into a tube of the present stripping catheter 20. As described, the tube (24 in FIG. 6) of the present stripping catheter 20 is possible not only to deliver a medicine but also to insert the guide wire 30.

The guide wire 30 has a shape in which an extreme end 30a in an inserting direction into a vein is curved, and is formed by a wire which is smaller in diameter than that of the tube of the catheter so as to facilitate passage into a vein blood vessel. The guide wire 30 extends through the tube in the stripping catheter 20 and projects from the extreme end of the insert head 22, and first moves into the vein blood vessel to guide the insertion of the stripping catheter 20. The reason why the extreme end 30a of the guide wire 30 is curved is to guide the stripping catheter 20 smoothly to a fixed place within the vein. In case of the stripping wire 1, in the insertion from the center portion of a vein to the peripheral portion, the vein meanders so that the resistance with respect to the stripping wire 1 is so great as to cause it to pass through slackening while performing a piston motion.

The guide wire 30 is inserted into the catheter whereby the stripping catheter 20 meandered within the blood vessel can be guided by the guide wire 30 and inserted into the peripheral portion of the blood vessel. With this, the vein can be inserted easily without passing through slackening on the skin while performing a piston motion.

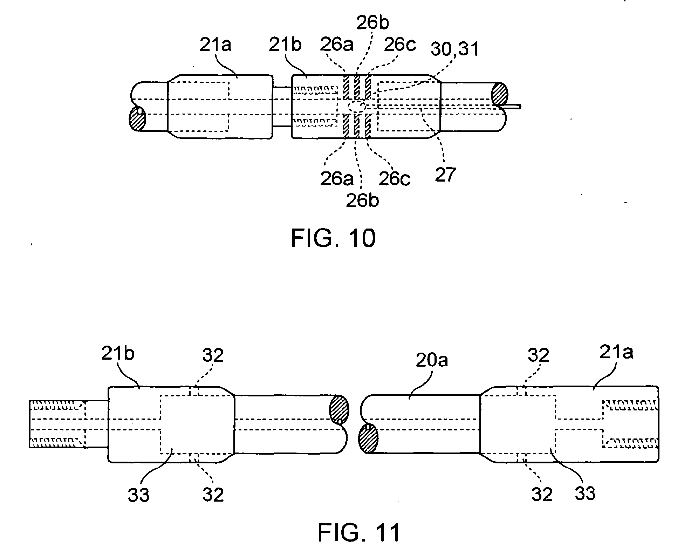

FIG. 10 shows the state that supersonic wave irradiating means 30 or laser beam irradiating means 31 is inserted into a tube of the present stripping catheter 20. As described, the tube (24 in FIG. 6) of the present stripping catheter 20 is also possible to insert the supersonic irradiating means 30 or the laser beam irradiating means 31 in place of the guide wire 30 described previously.

In FIG. 10, the supersonic wave irradiating means 30 or the laser beam irradiating means 31 irradiates supersonic waves or laser beam from a plurality of irradiation lights 26a, 26b and 26c provided in the second connecting means 21b. However, it is needless to say that it may be irradiated from the extreme end of the insert head 22 of the present stripping catheter 20. To a head for irradiating supersonic waves or laser beam of the supersonic wave irradiating means 30 or the laser beam irradiating means 31 is supplied power energy by a pair of lead wires inserted into a tube 27.

As described, for early recovery and treatment of the removed wound from which vein is introverted and removed, the laser beam or the supersonic waves is administered, before or after a medicine is administered into wound (affected part) by a medicine administering device or the like, to thereby recover and stop blood early within the vein removed wound.

FIG. 11 shows an example in which first connecting means 21a and second connecting means 21b are joined to a single catheter 20a.

As described above, the first connecting means 21a and the second connecting means 21b are joined to one end of the catheter 20a and the other end thereof, respectively. However, in the example shown in FIG. 11, the catheter 20a and the first and the second connecting means 21a and 21 b are joined by means of adhesives. Therefore, each of the connecting means 21a and 21b is provided with a depression 33 for receiving a catheter, and the end of the catheter 20a is inserted in the state that an adhesive is coated on the depression. The depression 33 is provided with a hole 32 directed toward the outer circumferential surface, thereby enhancing the adhesiveness relative to the catheter 20a.

As described above, in the stripping wire for introverting and removing a vein according to the present invention, it is possible that necessary number of a plurality of wires are connected for use to provide a stripping wire having a fixed length, and therefore this is able to cope with any diameter and length of veins. Further, not only the connecting portion to which a wire is connected but also the insert head or the rear end portion of the stripping wire become the vein ligating portion, thus enhancing the operating property and usefulness of the stripping wire.

The present invention has realized a stripping catheter for introverting and removing a vein and being provided with a tube for administering a medicine or the like. Also in the present stripping catheter, it is possible that necessary number of a plurality of wires are connected for use to provide a stripping catheter having a fixed length, and various shapes and sizes of insert heads prepared in advance can be selected adjusting to a vein subjected to introverting and removing to constitute a single stripping catheter.

### INDUSTRIAL APPLICABILITY

The present invention relates to a stripping wire for carrying out the removing operation of a vein or varicose veins used in the treatment site, and a stripping catheter for carrying out the removing operation of a vein or varicose veins and for pouring a medicine or the like into the affected part, which has an industrial applicability.

## Claims

1. A stripping wire for removing a vein, comprising:
a first wire having an insert head for guiding said stripping wire into a vein in one end thereof, and first connecting means in the other end thereof; and
a second wire having second connecting means that can be connected with said first connecting means in one end thereof, and a rear end portion in the other end thereof, wherein
said first wire and said second wire are connected for use, and there is formed a vein ligating portion in order that a connected portion caused by said first connecting means and said second connecting means, said insert head or said rear end portion is engaged with the affected part within the vein to introvert and remove the vein.

2. A stripping wire for removing a vein,
said stripping wire having a plurality of wires connected, each of said connected wires comprising first connecting means provided on one end thereof and second connecting means provided on the other end thereof, wherein
an insert head for guiding said stripping wire into a vein is connected to said first connecting means in one end of said connected wire,
a rear end portion is connected to said second connecting means in the other end of said connected wire, and
there is formed a vein ligating portion in order that a connected portion caused by said first connecting means and said second connecting means between the wires, said insert head or said rear end portion is engaged with the affected part within the vein to introvert and remove the vein.

3. A stripping wire according to Claim 1 or 2, wherein said first connecting means is formed from a fixed shaped male screw or female screw, and said second connecting means is formed from a male screw or a female screw in engagement with said first mentioned male screw or female screw.

4. A stripping wire according to Claim 2, wherein said insert head is provided with said second connecting means so that the former is connected to the first connecting means of said wire.

5. A stripping wire according to Claim 2, wherein said rear end portion is provided with said first connecting means so that the former is connected to the second connecting means of said wire.

6. A stripping wire for removing a vein according to Claim 1 or 2, wherein said insert head has an extreme end in an inserting direction formed into a convergent dome shape.

7. A stripping wire for removing a vein according to Claim 1 or 2, wherein said insert head is in an olive shape for removing a remained vein broken during removing the vein by a Babcock method.

8. A stripping wire according to Claim 1 or 2, wherein said rear end portion is in a cylindrical shape whose diameter is larger than said wire, and there is formed an operating grip for pushing in or drawing out in a direction of this side said insert head inserted into a vein.

9. A stripping wire according to Claim 1 or 2, wherein said wire is a twist wire of which outside diameter is 1.5 to 3.0 mm.

10. A stripping wire according to Claim 9, wherein the surface of said wire is applied with a coating of silicon resin or elastic synthetic resin.

11. A stripping wire for removing a vein according to Claim 10, wherein the surface of said wire is provided with depth marks at fixed intervals.

12. A stripping wire for removing a vein according to Claim 9, wherein the surface of said wire in the vicinity of said insert head is formed with fine concavo-convexes.

13. A stripping catheter for removing a vein, wherein said stripping catheter has a plurality of catheters connected, each of said catheters connected first connecting means provided on one end and second connecting means provided on the other end,
an insert head for guiding said stripping catheter into a vein is connected to said first connecting means on one end of said connected catheter,
a rear end portion is connected to said second connecting means on the other end of said connected catheter,
there is formed a vein ligating portion in order that a connected portion caused by said first connecting means and said second connecting means between the catheters, said insert head or said rear end portion is engaged with the affected part within the vein to introvert and remove the vein.

14. A stripping catheter according to Claim 13, wherein a tube (a tube hole) within said catheters connected extends through from said rear end portion to said insert head.

15. A stripping catheter according to Claim 14, wherein a pouring port for pouring a flowing medicine is provided in said rear end portion, and the medicine poured from said pouring port flows into a hypodermic tunnel after removal of a vein from a hole provided in said insert head or said connected portion.

16. A stripping catheter according to Claim 14, wherein a pouring port for pouring a flowing medicine is provided in said rear end portion, said catheter is formed with a plurality of holes from said tube extending through the interior to the circumferential surface of the catheter, and the medicine poured from said pouring port flows into a hypodermic tunnel after removal of a vein from said plurality of holes.

17. A stripping catheter according to Claim 15 or 16, wherein said pouring port is formed so that a syringe for pouring a medicine is connected.

18. A stripping catheter according to Claim 14, wherein a guide wire for guiding said catheter into a vein is introduced into the tube of said catheter connected.

19. A stripping catheter according to Claim 14, wherein supersonic wave irradiating means for irradiating supersonic waves to the affected part in a vein from a hole provided in said insert head or said connected portion is introduced into the tube within said catheter connected.

20. A stripping catheter according to Claim 14, wherein laser beam irradiating means for irradiating laser beams to the affected part in a vein from a hole provided in said insert head or said connected portion is introduced into the tube within said catheter connected.

21. A stripping catheter according to Claim 16, wherein a pouring port for pouring a flowing medicine is provided in the insert head, and after said insert head inserted into a vein is drawn outside the human body at a fixed place within the vein, the medicine is poured from the pouring port of said insert head.

22. A stripping catheter according to Claim 21, wherein in the connected portion between said a plurality of catheters, a closing portion for closing the tube extending through said catheter connected is formed.

23. A stripping catheter according to Claim 13, wherein said catheter is formed of a material whose main component comprises any of nylon, PTFE or polyimide - polyurethane.
